# FASCICULE DE BREVET EUROPEEN

(11) **EP 1 163 249 B1**
(45) Date de publication et mention de la délivrance du brevet: **07.12.2005**
(21) Numéro de dépôt: 00915270.3
(22) Date de dépôt: 31.03.2000
(51) Int. Cl.: C07H 15/207, A61K 7/48

(54) **BIOPRECURSEURS D'UN DERIVE RETINOIQUE ET COMPOSITIONS PHARMACEUTIQUES ET/OU COSMETIQUES**
BIOVORLÄUFER VON RETIN-DERIVATEN UND PHARMAZEUTISCH- UND/ODER COSMETIK-ZUSAMMENSETZUNGEN
BIOPRECURSORS OF A RETINOIC DERIVATIVE AND PHARMACEUTICAL AND/OR COSMETIC COMPOSITIONS

(30) Priorité: 31.03.1999 FR 9904032
(43) Date de publication de la demande: 19.12.2001
(73) Titulaire: Pierre Fabre Dermo-Cosmetique, 92100 Boulogne (FR)
(72) Inventeur: REDOULES, Daniel, F-31300 Toulouse (FR); TARROUX, Roger, F-31300 Toulouse (FR); FOURNIER, Didier, F-31320 Castanet Tolosan (FR); PERIE, Jean-Jacques, F-31320 Castanet Tolosan (FR)
(74) Mandataire: Ahner, Francis
(86) Numéro de dépôt international: PCT/FR2000/000822
(87) Numéro de publication internationale: WO 2000/058325

(56) Documents cités:
- WO-A-96/10403
- FR-A- 2 715 565
- DATABASE WPI Section Ch, Week 199834 Derwent Publications Ltd., London, GB; Class B03, AN 1998-393470 XP002122879 & JP 10 158290 A (NISSHIN OIL MILLS LTD), 16 juin 1998 (1998-06-16)
- REDOULES D ET AL: "Stereospecific Synthesis of Retinoic Acid glucoconjugates, as Pseudo-Substrates of Epidermal beta-Glucocerebrosidase" TETRAHEDRON LETTERS,NL,ELSEVIER SCIENCE PUBLISHERS, AMSTERDAM, vol. 40, no. 26, page 4811-4814 XP004168651 ISSN: 0040-4039

## Description

La présente invention se rapporte à une composition cosmétique ou pharmaceutique pour application cutanée, contenant un composé apte à libérer deux substances actives par action de deux activités enzymatiques, les activités glucocérébrosidase et estérase , ceci à partir d'un gluco-conjugué.

Il a été vérifié après sur-expression de la β-Glucocérébrosidase cutanée que cette enzyme était bien capable de reconnaître et d'hydrolyser de tels gluco-conjugués permettant ainsi un relargage lent de la substance active, sans effet d'accumulation.

La stratégie des bio-précurseurs a été précédemment utilisée pour la libération d'actifs dans deux cas précédents :
- libération de rétinol à partir de son ester avec l'acide palmitique sous l'action de l'activité esterase de la peau (J. Boenlein, et al. Characterization of esterase and alcohol dehydrogenase activity in skin. Metabolism of retinyl palmitate to retinol (Vitamin A) during percutaneous absorption. Pharm. Res. 11, 1155-1159 (1994).
- libération de vitamine C à partir d'un gluco-conjugué sous l'action dans ce cas d'une activité glucosidase (brevet FR-2 715 565).

Les dérivés rétinoïques sont aujourd'hui utilisés en dermatologie dans différentes indications comme le psoriasis ou ichtyose, ou bien pour obtenir une dépigmentation de la peau (réduction de la mélanogénèse sous l'action de la vitamine A) ; des applications contre le vieillissement de la peau sont également recherchées.

Ainsi, la demande japonaise JP 10 158 290 décrit la préparation du glucoside de rétinol et son utilisation dermatologique.

La demande internationale WO 96/10403 décrit des dérivés rétinoïques de formule générale : 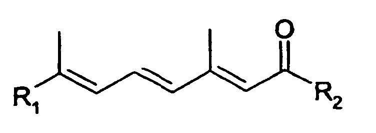 dans laquelle
R₁ = 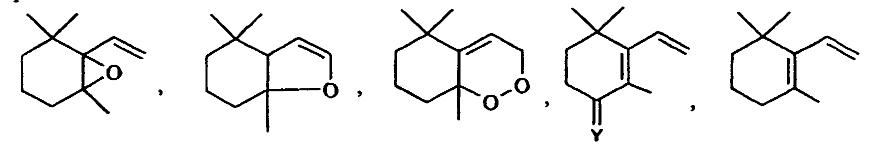 Y = O, H ou OH R₂ = OH, 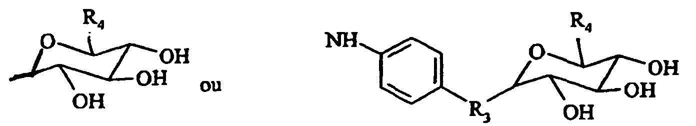 R₃ = O ou (CH₂)ₙ
n = 0 ou 1
R₄ = COOH ou CH₂OH

Cependant l'utilisation des dérivés rétinoïques par voie topique se heurte à un certain nombre de difficultés, du fait du manque de stabilité dans le temps et à la lumière de ces dérivés, de l'irritation résultant de sur-concentrations locales ainsi que d'une faible pénétration de ces dérivés au travers de la couche cornée. Ce dernier inconvénient est dû à la grande lipophilie de la substance qui déposée sur la peau est en fait en grande partie éliminée avec la desquamation. Par ailleurs, les effets secondaires (apparition de rougeurs, irritation, oedème et desquamation excessive) en limitent l'utilisation à des patients réellement motivés, tels ceux affectés d'une acné opiniâtre.

D'où l'intérêt de la présente invention d'amélioration de la bio-disponibilité de l'actif sous forme d'un complexe ternaire glucose-espaceur-actif, à pénétration facilitée et donc utilisable en faible quantité, évitant ainsi les effets néfastes de sur-concentrations locales, responsables des intolérances.

La présente invention concerne un complexe glucosylé ternaire, destiné à une application percutanée, de formule (I) 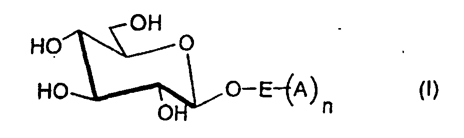 dans laquelle :
- E représente un groupement dérivé du glycérol L ou D, de l'hydroquinone ou de flavonoïdes,
- A représente un reste d'une molécule d'un principe actif rétinoïque; lié au groupement espaceur par une fonction carboxylate,
- n = 1 ou 2.

Le principe actif rétinoïque est avantageusement l'acide rétinoïque.

Selon une autre caractéristique de l'invention, dans le complexe de formule I, le groupement E représente un groupement doté d'une activité pharmaceutique et/ou cosmétique complémentaire, en particulier d'une activité d'hydratation; de dépigmentation, et/ou d'une activité antibactérienne.

En particulier, le groupement E peut représenter un groupement dérivé de flavonoïdes d'origine naturelle.

A titre d'exemple particulier de complexe glucosylé selon l'invention, on mentionnera :
- le para-rétinoyl-phényl-glucopyranoside,
- le dirétinoyl-1,2-propanyl-glucopyranoside,
- le rétinoate de daidzine, et
- le rétinoate de génistine.

La présente invention s'étend également à des compositions pharmaceutiques ou cosmétiques à usage topique, contenant un complexe glucosylé tel que défini précédemment, associé à un véhicule approprié pour l'administration percutanée.

Conformément à la présente invention, lorsque ladite composition est appliquée sur la peau, le complexe subit une double hydrolyse enzymatique, d'abord de type β-glucocérébrosidase conduisant à l'hydrolyse entre le glucose et le groupement espaceur, puis de type estérase conduisant à l'hydrolyse entre le groupement espaceur et le principe actif, ce dernier étant ainsi libéré de façon retardée sans effet d'accumulation dans les différentes couches de la peau.

Avantageusement, la composition selon l'invention contient de 0,001 à 10% en poids, de préférence de 0,01 à 0,1% en poids de complexe glucosylé par rapport au poids total de la composition.

La présente invention s'étend également à un procédé pour la préparation des complexes glucosylés précédemment définis, qui se caractérise en ce que l'on fait réagir un composé de formule II 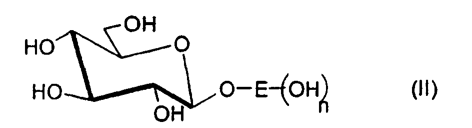 avec le principe actif sous forme de chlorure d'acide.

Selon une autre caractéristique de l'invention, le composé de formule II répond à la formule plus précise IIa suivante

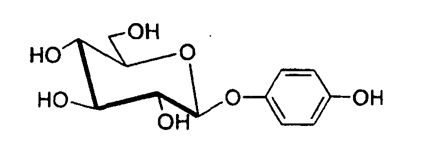

Selon une autre caractéristique, le procédé de formule II répondant à la formule IIb suivante

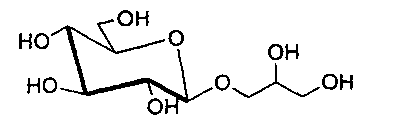

Enfin selon une dernière caractéristique de l'invention, le procédé implique la réaction entre les composés de formule II, IIa ou IIb avec le chlorure de rétinoyle.

Le complexe glucose-espaceur-actif, après rapide migration dans les premières couches de l'épiderme du fait de son caractère d'amphiphile est reconnu en tant que pseudo-substrat par les deux activités enzymatiques impliqués : β-glucocérébosidase (EC 3.2.1.45) responsable de l'hydrolyse entre glucose et espaceur, puis esterase responsable de la seconde hydrolyse entre espaceur et actif. Bien entendu l'espaceur peut lui même être choisi en tant qu'actif : ceci est réalisé ici en utilisant l'hydroquinone comme espaceur, lui même actif en tant que dépigmentant, ou antibactérien. Deux effets conjugués sont ainsi obtenus à partir d'une formulation unique.

Il a été démontré que les gluco-conjugués décrits dans l'invention permettent une réelle stabilisation des actifs rétinoïques ainsi qu'une très bonne pénétration : alors que des dérivés trop lipophiles comme l'acide rétinoïque ou la vitamine E (α-tocophérol) s'accumulent dans les couches supérieures du stratum cornéum après application topique et sont éliminés par desquamation, leurs gluco-conjugués au contraire inclus dans un même excipient, sont retrouvés pour partie (partie non encore hydrolysée) au sein des couches supérieures et aussi inférieures du stratum cornéum, et ceci plusieurs jours après leur application.

La conception de ces gluco-conjugués en tant que pseudo-substrats dirigés vers l'activité β-Glucocérébrosidase pour la première hydrolyse est justifiée par plusieurs facteurs :
- cette enzyme est accessible depuis la surface cutanée comme cela a été montré par application topique d'un inhibiteur spécifique (W. M ; Holleran, P. M Elias. J. Lipid. Res. 1994, 35. 905) ;
- cette activité enzymatique prépondérante dans la formation des lipides de la surface cutanée (40% des lipides résultent de cette activité) est bien conservée d'une par entre sujets et d'autre part au cours du cycle des saisons ;
- dans les conditions utilisées dans la présente invention, cette activité est suffisante, puisque supérieure à l'activité estérase (exemple 1).

Cette enzyme a donc été sur-exprimée ; cela a permis de déterminer les paramètres cinétiques des substrats par rapport à une référence. Des valeurs sont données à titre d'exemple pour 2 conjugués, l'un à 2 l'autre à 3 composants. Les valeurs indiquent que ces pseudo-substrats sont mieux reconnus que le substrat de référence (valeurs des K_{M}), ce qui s'explique par le caractère plus lipophile de ces conjugués par rapport à la référence 4-methylumbellifery-glucopyranoside vis à vis d'une enzyme dont le substrat est lui même très lipophile (β-glucosyl-ceramide) ; d'autre part les valeurs Vₘ, montrent que les actifs sont bien relargués et ceci avec des cinétiques compatibles avec l'objectif visé, à savoir un effet dans le temps à partir d'un pseudo-substrat appliqué sur la peau en quantité minimale mais qui sera intégralement utilisée.

La stratégie présentée précédemment peut être étendue et modifiée dans différentes directions. A titre d'exemples :
- *modification de l'espaceur :*
   Celui-ci peut être modifié en une structure plus proche de celle du substrat naturel (β-Glucocérébroside) dans lequel l'espaceur est apparenté au glycérol. Le glucoconjugué correspondant glucose-glycérol (L ou D) - acide rétinoïque a également été synthétisé et étudié. Notons que les deux groupements hydroxyle libres sur le glycérol permettent de fixer sous forme d'ester deux unités rétinoïques par molécule de complexe. Dans ce cas de figure, l'action complémentaire à l'activité rétinoïque est celle d'un effet hydratant apporté par la libération in situ du glycerol ;
- *association à l'activité rétinoïque de propriétés anti-oxydantes de flavonoïdes :*
   Un certain nombre de flavonoïdes d'origine naturelle sont associés à une partie saccharidique qui leur confère des propriétés d'amphiphiles.
   C'est par exemple le cas des génistines ou de la daidzine. L'absorption de tels composés par la surface cutanée est donc assurée. A cette première activité d'anti-oxydant, est associée l'activité rétinoïque par fixation d'une ou plusieurs molécules d'acide rétinoïque par unité flavone. Les structures correspondantes sont présentées ci-après :

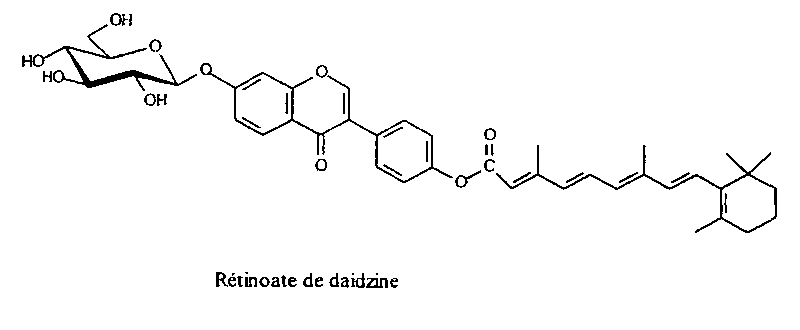

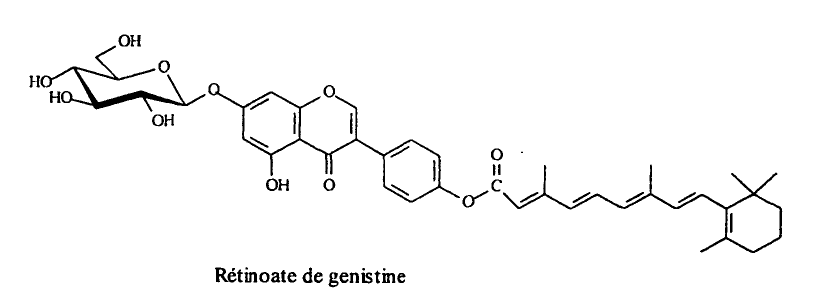

En conclusion, la présente invention montre le parti qu'il peut être pris des activités β-Glucocérébrosidase et esterase de la surface cutanée pour obtenir la libération de différents types d'actifs à partir de bio-précurseurs glucosylés.

La structure des gluco-conjugués correspondants assure, une bonne pénétration en raison de leur caractère d'amphiphile et donc une utilisation optimale, une très bonne reconnaissance par la première enzyme β-glucocérébrosidase du fait de la présence d'un ou plusieurs résidus rétinyl lipophiles et une libération des actifs avec une cinétique assurant une coupure effective et un effet avec rémanence dans le temps.

Les synthèses des gluco-conjugués, leur formulation ainsi que leur activité en tant que pseudo-substrats sont décrits ci-dessous :

### a) Synthèse des bioprécurseurs

Le rétinoate d'arbutine (p-rétinoyl-phényl-glucopyranoside) est préparé à partir de l'arbutine et du chlorure de rétinoyl selon le schéma réactionnel suivant. 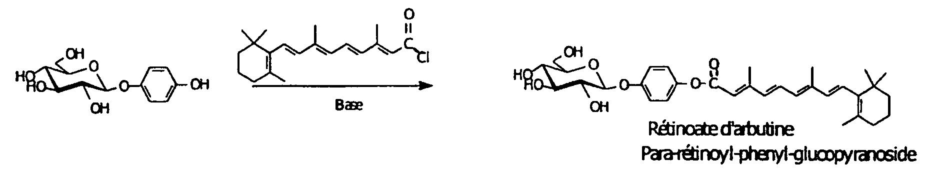 Cette réaction de couplage résulte d'une déshydrogénation sélective et initiale de la fonction phénol suivie d'une attaque nucléophile du phénoxy formé sur le chlorure d'acide. La déshydrogénation sélective est obtenue par l'addition, au maximum, d'un équivalent de base (généralement 0.9 équivalent) réagissant sur le groupement phénol (pKa=9) de pKa bien plus faible que les autres fonctions hydroxyle de la partie glucose (pKa > 16).

### Préparation du chlorure de rétinoyl

A une suspension de 1g (3.32 mmol) d'acide rétinoique dans 15 ml de chlorure de méthylène anhydre refroidie à 0°C, maintenue sous argon et contenant 0.32g de pyridine (0.4 mmol), on ajoute, goutte à goutte, 0.41 g (3.3 mmol) de chlorure de thionyle dans le chlorure de méthylène (2 ml). On laisse revenir à température ambiante et on poursuit l'agitation pendant 1 heure. On filtre sur laine de verre le sirop rouge obtenu qui est immédiatement utilisé dans l'étape suivante.

### Préparation du rétinoate d'arbutine (p-rétinoyl-phényl-glucopyranoside)

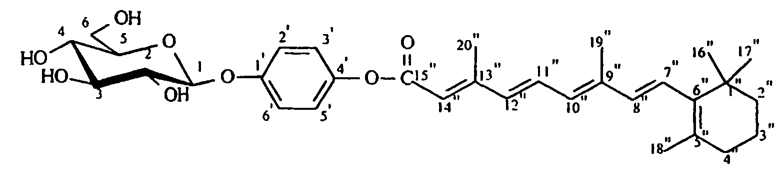

A une suspension de 50 mg (2.1 mmol) d'hydrure de sodium dans 10 ml de DMF anhydre refroidie à 0°C et maintenue sous argon, on additionne, goutte à goutte, 0.7 g (2.6 mmol) d'arbutine. On ajoute lentement les 15 ml de la solution de chlorure de rétinoyl préparés précédemment et on agite le mélange pendant 1 heure en laissant revenir à température ambiante. On hydrolyse l'excès de chlorure d'acide par 5 ml d'eau et on neutralise par ajout de quelques gouttes d'une solution saturée d'hydrogénocarbonate de sodium. La phase organique extraite, séchée et évaporée sous vide est purifiée par HPLC (C18 : MeOH-H₂O : 85-15).

On obtient 1.1 g de cristaux rouges. Rd= 73 %.
RMN ¹H (CDCl₃) δ ppm :
6.9-7.1 (m,5H, H-2', 3', 5', 6', 11"), 6.1-6.35 (m, 4H, H-7", 8" -CH=CH, 10", 12" -CH=CH), 5.88 (s, 1H, H-14" -CH=CH-), 4.84 (d, 1H, H-1), 3.3-3.9 (m, 6H, H-2, 3, 4, 2 H6), 2.3 (1 s, 3H, H-20" -CH₃), 1.97-2.03 (1s et m, 5H, H-19" -CH₃, 4" -CH₂), 1.36-1.68 (1m, 1s, 7H, H-2", 3" -(CH₂)₂, 18" -CH₃), 1. 02 (1s, 6H, H-16", 17" -CMe₂).
RMN ¹³C (CDCl₃) δ ppm :
166 (C-15"), 155.5 (C-13"), 159.7 (C-1'), 145.6 (C-4'), 140.2 (C-9"), 137.7 (C-6"), 137.4 (C-8"), 135.1 (C-12"), 131.9 (C-11"), 130 (C-5"), 129.7 et 128.9 (C-10", 7"), 122.8 (C-3', 5'), 117.8 (C-14"), 117.4 (C-2',6'), 100.1 (C-1), 75.7 (C-3), 75 (C-5), 71.5 (C-2), 70.1 (C-4), 61.7 (C-6), 39.6 (C-2"), 34.3 (C-1"), 33.2 (C-4"), 29 (C-16", 17"), 21.8 (C-18"), 19.3 (C-3"), 14.1 et 13 (C-20", 19")
IR : 3418 cm⁻¹ OH, 1700 cm⁻¹ (ester C=O), 1684, 1576, 1504, 1447, 1358, 1195, 1129 cm⁻¹ (CO)
SM (m/z) 555 (M⁺+1), 577 (M⁺+Na).

Dans le but d'étudier la cinétique de coupure du rétinoate d'arbutine par la βglucocérébrosidase, nous avons synthétisé le produit de son hydrolyse : le p-rétinoate de 4-hydroxyphenyle.

### Préparation du p-rétinoate de phénol

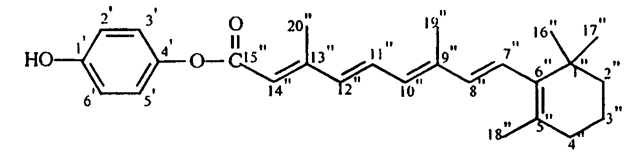

On ajoute 300 mg de Na₂CO₃ (2.8 mmol) séchés à une solution d'hydroquinone (300 mg, 2.7 mmol) dans l'acétone anhydre (15 ml) maintenue sous argon, puis lentement les 15 ml de la solution de chlorure de rétinoyl (max 3 mmol) préparé précédemment. Après 1 heure d'agitation, on hydrolyse le chlorure d'acide en excès en ajoutant 5 ml d'eau et on neutralise le milieu par ajout de quelques gouttes d'une solution saturée de NaHCO₃. La phase organique extraite, séchée, évaporée sous vide et purifiée par HPLC (C₁₈ : éluant MeOH-H₂O : 90-10) fournit 0.61 g de cristaux rouges (Rdt = 52 %).
RMN ¹H (CDCl₃) δ ppm :
6.95 et 6.78 (2d, 4H, H-2', 3', 5', 6', J = 11Hz), 7.07 (dd, 1H, H-11"), 6.1-6.4 (m, 4H, H-7", 8" -CH=CH, 10", 12" -CH=CH), 5.8 (s, 1H, H-19" -CH=CH-), 2.4 (1 s, 3H, H-20" -CH₃), 2-2.1 (1s et m, 5H, H-19" -CH₃, 4" -CH₂), 1.4-1.72 (1m, 1s, 7H, H-2", 3" -(CH₂)₂, 18" -CH₃), 1.02 (1s, 6H, H-16", 17" -CMe₂).
RMN ¹³C (CDCl₃) δ ppm :
166.4 (C-15"), 155.3 (C-13"), 153.8 (C-1'), 143.9 (C-4'), 140.3 (C-9"), 137.7 (C-6"), 137.4 (C-8"), 135 (C-12"), 131.9 (C-11"), 130.2 (C-5"), 129.5 et 128.9 (C-10", 7"), 122.8 (C-3', 5'), 117.8 (C-14"), 117.3 (C-2',6'), 39.6 (C-2"), 34.3 (C-1"), 33.2 (C-4"), 29 (C-16", 17"), 21.8 (C-18"), 19.3 (C-3"), 14.2 et 13 (C-20", 19")
SM (FAB/ MNBA) m/Z : 415 (M⁺+Na).

### Synthèse du dérivé dirétinoyl-1,2-propanylglucopyranoside (conjugué glucose-glycérol-acide rétinoique)

La figure ci-dessous décrit le schéma réactionnel que nous avons emprunté pour réaliser la synthèse des composés **6** et **7** (énantiomères en C₂ de l'espaceur glycérol). 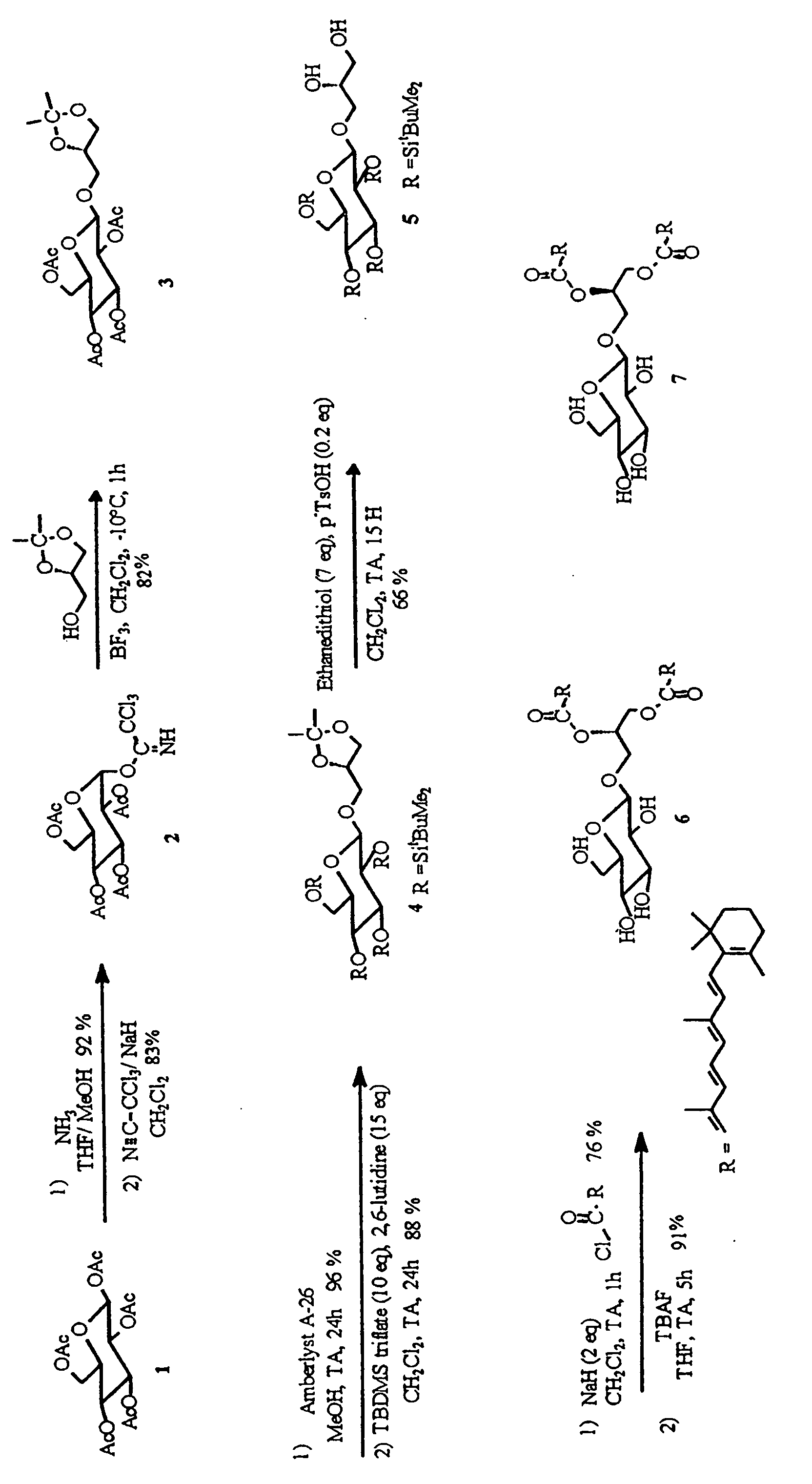 La déacylation sélective en position 1 a été obtenue par aminolyse du glucopyranose peracétylé 1 en mettant en oeuvre l'ammoniac dans le mélange (THF-MeOH : 7-3).

Le gluco-conjugué 3 a été préparé selon la méthode de Schmidt (Schmidt, R.R. Angew. Chem. Int. Ed. Engl. 1986, 25, 212) qui permet un couplage stéréosélectif en utilisant l'imidate comme activateur nucléofuge.

Cet intermédiaire est synthétisé par action de l'hydrure de sodium sur le glucopyranose déprotégé en C-1, qui tranformé en alkoxyde, réagit comme nucléophile sur le trichloroacétonitrile pour donner l'α-imidate 2.

Le spectre IR de ce composé présente la bande caractéristique à 1670cm⁻¹ attribuable à la liaison imine C=N Le spectre RMN ¹H de ce composé comporte un doublet à 6.6 ppm qui traduit la présence de l'hydrogéne en 1 couplé à l'hydrogène sur le carbone C-2, dans une configuration α (J = 3.5 Hz).

En présence d'acide de Lewis (BF3 etherate), l'α-imidate tétraacétylé 2 réagit avec un alcool dans le chlorure de méthylène et conduit à la formation du glucoconjugué correspondant. Cette réaction résulte d'une activation initiale de la fonction imidate par l'acide de Lewis, suivie d'une attaque nucléophile de l'alcool sur le carbone 1 de la partie osidique pour donner exclusivement le dérivé β-glucosylé (J=8Hz en C₁).

La déprotection des glucoconjugués tétraacétylés est obtenue par traitement par résine échangeuse d'ions (amberlyst A-26 (OH) selon une série d'échanges ioniques à la surface de la résine.

Une filtration rapide après une nuit de contact avec la résine permet d'isoler facilement avec un rendement important le composé hydrosoluble déprotégé.

La silylation des dérivés osidiques par le TBDMS triflate ne conduisant généralement qu'à de très faibles rendements (T. Limori, H. Takashashi and S. Ikegami, Tetrahedron Lett., 1996, 37, 649), nous avons mis au point les conditions d'obtention d'une silylation des 4 fonctions hydroxyle libres du glucopyranose. La structure du dérivé obtenu est établie par le spectres de RMN ¹H : la présence des protons méthyliques des groupes TBDMS et leur intégration établit avec certitude la tétrasilylation.

L'hydrolyse sélective de l'acétal 4 sans le départ concomitant des protections silyl a pu être obtenue avec un rendement de 66 % en utilisant un excès d'éthane-dithiol en présence d'une quantité catalytique d'acide p-toluène-sulfonique dans le chlorure de méthylène. La structure du composé 5 est déduite des spectres IR (bande OH à 3390 cm⁻¹ et de masse (FAB M⁺+ Na= 733), des spectres de RMN du proton et du ¹³C qui montrent la disparition des méthyles de l'acétal.

La double estérification est obtenue avec 76 % de rendement, selon la méthode appliquée précédemment. Ainsi, en présence de deux équivalents d'hydrure de sodium, le diol réagit avec le chlorure de rétinoyl pour donner le di-ester attendu. Les caractéristiques spectrales sont conformes à la structure proposée. Les spectres RMN ¹H et ¹³C montrent la présence des synthons rétinoiques et glucose tétrasilylé.

L'étape finale de déprotection des groupes hydroxyle portés par le motif saccharidique a été ensuite pratiquée dans du THF anhydre ,en présence de 4 équivalents de TBAF et conduit au conjugué glucose-glycérol-acide rétinoique 6 avec un rendement de 90 %.

(TBDMS = tert-butyldimethylsilyl ; TBAF = tetra-n-butylammonium fluoride))

### Préparation du dérivé 3

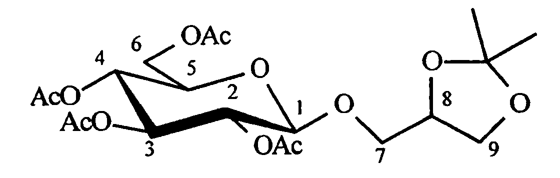

On ajoute lentement, 100 mg d'éthérate de BF₃ en solution dans 1 ml de CH₂Cl₂ à un mélange refroidi à -10°C de 1.6g d'imidate (4.6 mmol) et de 0.6g d'α, β-isopropylidèneglycérol (4.6 mmol) dans 30 ml de CH₂Cl₂. On maintien l'agitation pendant 2h, on lave avec NH₄Cl saturé et on neutralise avec une solution saturée de NaHCO₃. Après séchage (MgSO₄), on concentre sous pression réduite et on purifie le résidu brut par chromatographie flash (éluant : hexane-acétate d'éthyle : 3-2). On obtient 1.74g (3.8 mmol) de cristaux blancs.
RMN ¹H CDCl₃ δ ppm (300 MHz) :
4.36-5.19 (m, 3H, H-1, 2, 3), 4.59(dd, 1H, H-5), 4.23-3.57 (m, 8H, H-4, 8, 2H6, 2H7, 2H9), 1.96-2.07 (4s, 12H, Ac), 1.39 et 1.32 (2s, 6H, CH₃ acétal).
RMN ¹³C (CDCl₃) δ ppm :
169.3-170.7 (4s, 4 OCOR), 109.4 (C_{quat}, isopropylidène), 101 (C-1), 74.2 (C-8), 72.8 (C-3), 71.9 (C-5), 71.2 (C-2), 69.2 (C-7), 68.4 (C-4), 66.8 (C-9), 61.9 (C-6), 26.6 et 25.4 (2CH₃ des acétals.
IR : 1756 cm⁻¹ (ester C=O), 1370, 1229,1167, 1050 cm⁻¹ (CO)

### Préparation du dérivé silylé 4

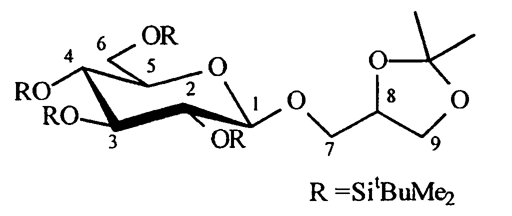

On laisse pendant 24 heures à température ordinaire, une solution de 400 mg (0.86 mmol) du gluco-conjugué 3 dans 20 ml de MeOH contenant 75 mg de résine Amberlyst A26. La solution filtrée et concentrée fournit 250 mg de dérivé glucopyranoside déprotégé (0.85 mmol).

Une solution du dérivé déprotégé précédent (250 mg) contenant 1.1g de lutidine (10 mmol) dans 15 ml de chlorure de méthylène anhydre, refroidi à 0°C et sous argon, est additionnée de 1.8g (6.8 mmol) de TBDMS triflate. Le mélange est maintenu sous agitation à température ordinaire pendant 30 heures. La solution organique lavée, séchée et évaporée sous vide fournit après purification par chromatographie flash, 0.4 g de résine incolore (éluant : Hexane-AcOEt : 30-1).
RMN ¹H CDCl₃ δ ppm (300 MHz) :
4.68 (d, 1H, H-1, Jaa=10 Hz), 4.32 (dd, 1H, H-3), 4.05 (t, 1H, H-8), 3.58-3.89 (m, 9H, H-2, 4, 5, 2H6, 2H7, 2H9), 1.35 et 1.41 (2s, 6H, CH₃ acétal), 0.85-0.9 (4s, 36H, 4 Si^{t}Bu), 0.04-0.09 (4s, 24H, SiMe₂).
RMN ¹³C (CDCl₃) δ ppm :
109 (C_{quat}, isopropylidène), 102.3 (C-1), 82.4 (C-3), 79.1 (C-5), 77.5 (C-2), 79.5 (C-8), 70.2 (C-4), 70.1 (C-7), 67.6 (C-9), 64.2 (C-6), 26.9 et 25.5 (2CH₃ des acétals), 25.9 (CH₃ (^{t}Bu)) 17.9-18.4 (4s, C_{quat}-Si), -4.11-(-5.4) (4s, CH₃Si).
SM (FAB/ ONPOE) m/z : 773 (M⁺+Na)
IR : 1472, 1361, 1255, 1096 cm⁻¹ (CO)

### Préparation du dérivé 5

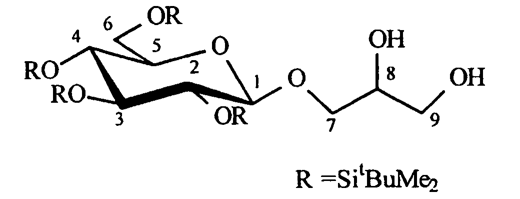

A une solution de 1g de 4 (1.33 mmol) dans 20 ml de chlorure de méthylène, on ajoute sous argon et sous agitation mécanique 0.88g d'éthane dithiol (9.33 mmol) et 25 mg d'acide p-toluène sulfonique (0.132 mmol). On maintient l'agitation pendant 15 heures encore. Après lavage avec une solution saturée de NaCl, séchage (MgSO4) puis filtration, on récupère après concentration sous vide un résidu qui est purifié par chromatographie flash (hexane-acétate d'éthyle : 1-1). On recueille ainsi 0.625g d'huile incolore (Rdt = 66 %).
RMN ¹H CDCl₃ δ ppm (300 MHz) :
4.67 (d, 1H, H-1, Jaa=10 Hz), 3.53-3.98 (m, 13H, H-2, 3, 4, 5, 2H6, 2H1', 2H2', 2H3', 2 OH), 0.85-0.9 (4s, 36H, 4 Si^{t}Bu), 0.038-0.09 (4s, 24H, SiMe₂).
RMN ¹³C (CDCl₃) δ ppm :
103.3 (C-1), 82.7 (C-3), 78.9 (C-5), 78.2 (C-2), 72.2 (C-1'), 71 (C-4), 70.2 (C-2'), 64.2 (C-6), 63.9 (C-3'), 25.9 (CH₃ (^{t}Bu)), 18.4-17.9 (4s, C_{quat} Si), -4.11-(-5.4) (4s, CH₃Si).
SM (FAB/ ONPOE) m/z : 733 (M⁺+Na)
IR : 3390 cm⁻¹ (OH), 1384, 1218, 1078 cm⁻¹ (CO)

### Préparation du dérivé 6 (S)ou 7 (R)

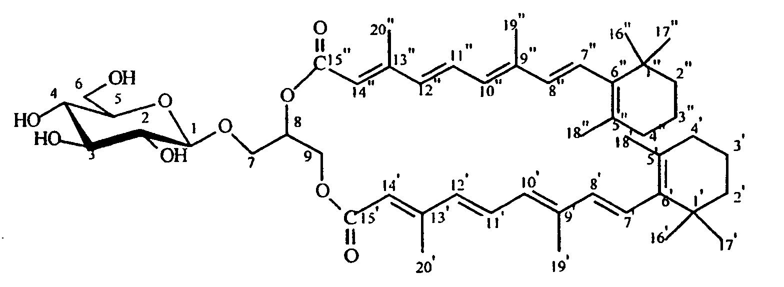

La double estérification est conduite selon le mode opératoire décrit pour la synthèse du rétinoate d'arbutine, mais ici nous prenons le chlorure de méthylène comme solvant et utilisons 2 équivalents d'hydrure de sodium. La séparation du composé estérifié qui se trouve dans la zone Rf=0.2 élué par le mélange (Hexane-AcOEt : 25-1) est réalisée par chromatographie flash.

On désilyle le diester obtenu (0.68g, 0.53 mmol) par 2.3 g de TBAF (7.4 mmol) dans 15 ml de THF anhydre. Après 4 h d'agitation, lavages de l'extrait et évaporation à sec, on purifie par CCM sur gel de silice, type 60, dans un mélange CH₂Cl₂-MeOH (95-5) Rf = 0,3. On isole 0,4 g de cristaux rouges.
(6, α_{D}= - 8°, forme S)
(7, α_{D}= + 12°, forme R)
RMN ¹H CDCl₃ δ ppm (300 MHz) :
6.97 (dd, 2H, H-11',11"C=CH, JJ= 16Hz), 6.08-6.3 (m, 8H, H-7', 7", 8', 8"-HC=CH, H-10',10", 12', 12" -C=CH), 5.74 (s, 2H, H-14', 14"-CH=CH,), 4.32-4.37 (m,2H, H-1,8), 3.23-3.96 (m, 14H, H-2, 3, 4, 5, 2H6, 2H7, 2H9), 2.3 (s, 6H, H-20', 20" -CH₃), 1.97-2.03 (1s et m, 10H, H-19' , 19" -CH₃, H-4', 4'' -CH₂), 1.36-1.68 (1m, 1s, 14H, H-2', 3' , 2", 3" - (CH₂)₂, H-18', 18" -CH₃), 0.94, 0.98, 1, 1.01, (4s, 12H, H-16', 16", 17', 17" -CMe₂).
RMN ¹³C (CDCl₃) δ ppm :
167, 166.5 (C-15', 15"), 154.3, 153.8 (C-13', 13") , 140 (C-9', 9"), 137.7 (C-6,' 6"), 137.3 (C-8, 8"), 135.1 (C-12', 12"), 131.6 (C-11', 11"), 130.4 (C-5', 5"), 129.6 et 128.8 (C-10', 10", 7', 7"), 117.9 (C-14', 14"),103.7 (C-1), 76.1 (C-8), 73.7 (C-3,5), 70 (C-2,4), 68.3 (C-7), 62.5 (C-9), 62 (C-6), 39.6 (C-2', 2"), 34.3 (C-1', 1"), 33.2 (C-4', 4"), 29 (C-16', 16", 17', 17"), 21.8 (C-18', 18"), 19.3 (C-3', 3"), 13.8 et 13 (C-20', 20", 19', 19")
IR : 3427 cm⁻¹ OH, 1706 cm⁻¹ (ester C=O), 1609, 1457, 1384, 1237, 1141, 1083 cm⁻¹ (CO)
SM (FAB/ MNBA) m/z : 841 (M⁺+Na)

### b) Formulations

Les compositions selon l'invention contiennent de 0,001 à 10 % en poids, de préférence 0,01 % à 0,1% en poids, de précurseurs d'actifs par rapport au poids total de la composition.

La composition selon l'invention peut se présenter sous forme d'émulsion huile dans eau (H/E) ou eau dans huile (E/H). Elle peut encore se présenter sous forme de sphérules comme les liposomes, les nanocapsules ou les nanosphères.

Lorsque la composition est une émulsion, la proportion de la phase grasse va de 5 à 80 % en poids, de préférence de 5 à 50 % en poids, par rapport au poids total de la composition. Les huiles, les émulsionnants et les coémulsionnants utilisés dans la composition, sous forme d'émulsion, sont choisis parmi ceux classiquement utilisés en cosmétique. L'émulsionnant et le coémulsionnant sont présents, dans la composition, en une proportion allant de 0.3 à 10 % en poids, par rapport au poids total de la composition.

La composition selon l'invention peut également contenir des additifs cosmétiques ou dermatologiques acceptables. Ces additifs peuvent être, en particulier, des antioxydants, des bioprécurseurs de ces antioxydants comme le δ-tocophérylglucopyranoside, des tensioactifs, des corps gras, des agents hydratants, des conservateurs, des parfums, des gélifiants, des chélateurs, des pigments comme l'oxyde de titane, des filtres et des vitamines libres comme l'acide ascorbique.

### c) Etude enzymatique

### - Comparaison des activités β-glucocérébrosidase et estérase

La technique de stripage permet de doser avec une précision très satisfaisante ces deux activités distinctes à partir d'un même prélèvement. Nous avons utilisé, pour cela, deux substrats artificiels, le 4-méthyl-umbelliferyl-β-D-glucopyranoside (2 mM) pour le dosage de l'activité β-glucocérébrosidase et le 4-méthyl-umbelliferyl-palmitate (2 mM) pour celui des estérases.

Le tableau suivant donne la quantité de 4-méthylumbelliferone libérée suite à l'hydrolyse en 1 heure par les β-glucocérébrosidase et estérase extraites de trois stripages de 25 cm².

On note qu'à pH cutané (pH = 5.5), l'activité β-glucocérébrosidase est en moyenne deux fois plus élevée que celle de l'estérase.

| | β-glucocérébrosidase | Estérase |
|---|---|---|
| Activités pondérées Nmoles/ heure/ µg de protéines totales | 0,23 ± 0,1 | 0,13 ± 0,08 |

### - Reconnaissance et hydrolyse des pseudo-substrats

Après avoir vérifié que la β-glucocérébrosidase est exprimée dans les kératinocytes, nous avons produit une enzyme recombinante dans le système baculovirus. Une queue histidine a été rajoutée à l'extrémité COOH de la protéine pour permettre sa purification par chromatographie sur colonne d'affinité.

Ainsi, nous avons pu déterminer les constantes de Michaelis (Km) et les Vm de la β-glucocérébrosidase recombinante pour notamment le gluco-conjugué acide rétinoique arbutine. Les mesures de cinétique sont effectuées dans un tampon phtalate à pH 5.6 (0.025 M) contenant du taurocholate (5 mM), de la β-glucocérébrosidase purifiée et le conjugué étudié aux différentes concentrations. L'incubation dure 30 minutes et le dosage de la quantité du conjugué hydroquinone-acide rétinoique libéré est réalisé par HPLC. Le tableau ci-dessous donne les résultats obtenus. Ils montrent en termes d'affinité que les deux glucoconjugués étudiés sont bien meilleurs substrats que la référence. En ce qui concerne leur vitesse d'hydrolyse, elle est inférieure et permet donc d'obtenir des effets dans le temps.

| Substrats | Km | Vm pondérée par la quantité de protéines solubles. |
|---|---|---|
| 4-Méthylumbelliférylglucopyranoside | 2,8 ± 0,7mM | 4000 ± 1000 nmoles/ h/ mg |
| δ-Tocophérylglucopyranoside | 7 ± 1 µM | 453 ± 20 nmoles/ h/ mg |
| Rétinoate d'arbutine | 5 ± 1,2 µM | 235 ± 19 nmoles/ h/ mg |
| Dirétinyl-glycérylglucopyranoside 7 (R) | 8,6 ± 2,5 µM | 74 ± 7 nmoles/h/mg |
| Dirétinyl-glycérolglucopyranoside 7 (S) | 5 ± 0,4 µM | 17 ± 0,4 nmoles/h/mg |

## Revendications

1. Complexe glucosylé ternaire, destiné à une application percutanée, de formule (I) 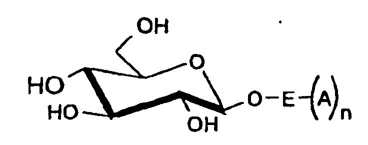 dans laquelle :
- E représente un groupement dérivé de glycérol L ou D, de l'hydroquinone, ou de flavonoïdes,
- A représente un reste d'une molécule d'un principe actif rétinoïque, lié au groupement espaceur par une fonction carboxylate -O-C(O)-,
- n = 1 ou 2.

2. Complexe glucosylé selon la revendication 1, **caractérisé en ce que** le principe actif rétinoïque est l'acide rétinoïque.

3. Complexe glucosylé selon l'une des revendications 1 à 2, **caractérisé en ce que** le groupement E représente un dérivé de flavanoïdes d'origine naturelle.

4. Complexe glucosylé selon l'une des revendications 1 à 3, **caractérisé en ce qu'**il est choisi parmi :
- le para-rétinoyl-phényl-glucopyranoside,
- le dirétinoyl-1,2-propanyl-glucopyranoside,
- le rétinoate de daidzine, et
- le rétinoate de génistine.

5. Composition pharmaceutique ou cosmétique à usage topique, **caractérisée en ce qu'**elle contient un complexe glucolysé selon l'une des revendications 1 à 4, associé à un véhicule approprié pour l'administration percutanée.

6. Composition selon la revendication 5, **caractérisée en ce qu'**elle contient de 0,001 à 10 % en poids, de préférence 0,01 à 0,1% en poids, de complexe glucosylé par rapport au poids total de la composition.

7. Composition selon l'une des revendications 5 à 6 , **caractérisée en ce qu'**elle se présente sous forme d'émulsion.

8. Composition selon l'une des revendications 5 à 6, **caractérisée en ce qu'**elle se présente sous forme de sphérules, comme les liposomes, les nanocapsules ou les nanosphères.

9. Procédé de préparation d'un complexe selon l'une des revendications 1 à 4, **caractérisé en ce que** l'on fait réagir un composé de formule (II) 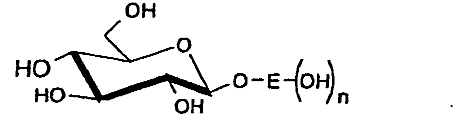 avec le principe actif rétinoïque sous forme de chlorure d'acide.

10. Procédé selon la revendication 9, **caractérisé en ce que** le composé de formule II répond à la formule suivante IIa :

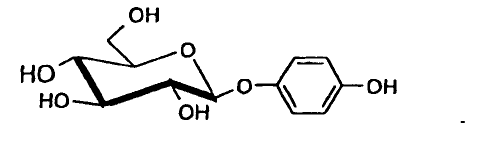

11. Procédé selon la revendication 9, **caractérisé en ce que** le composé de formule II répond à la formule suivante IIb :

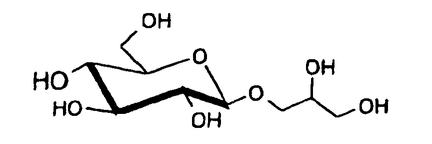

12. Procédé selon l'une des revendications 9 à 11, **caractérisé en ce que** le chlorure d'acide est le chlorure de rétinoyle.

13. Utilisation d'une composition selon l'une des revendications 5 à 8, pour la préparation d'un médicament ou d'une composition cosmétique destinée à, lorsqu'il est appliqué sur la peau, libérer de façon retardé le principe actif rétinoïque de façon retardé sans effet d'accumulation dans les différentes couches de la peau, par double hydrolyse enzymatique du complexe, d'abord de type β-glucocérébrosidase conduisant à l'hydrolyse entre le glucose et le groupement espaceur, puis de type estérase conduisant à l'hydrolyse entre le groupement espaceur et le principe actif,

14. Utilisation selon la revendication 13, **caractérisée en ce que** le groupement E est doté d'une activité pharmaceutique et/ou cosmétique.

15. Utilisation selon l'une des revendications 13 à 14, **caractérisée en ce que** le groupement E est doté d'une activité d'hydratation, de dépigmentation et/ou antibactérienne.

## Patentansprüche

1. Ternärer glucosylierter Komplex, welcher für eine perkutane Anwendung bestimmt ist, der Formel (I) 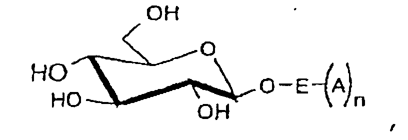 in welcher:
- E für eine von L- oder D-Glycerol, von Hydrochinon oder von Flavonoiden abgeleitete Gruppierung steht,
- A für einen Rest eines Moleküls eines retinoischen Wirkstoffs, der mit der Abstandshaltergruppierung durch eine Carboxylatfunktion -O-C(O)- verbunden ist, steht,
- n = 1 oder 2.

2. Glucosylierter Komplex nach Anspruch 1, **dadurch gekennzeichnet, dass** der retinoische Wirkstoff Retinsäure ist.

3. Glucosylierter Komplex nach einem der Ansprüche 1 bis 2, **dadurch gekennzeichnet, dass** die Gruppierung E für ein Derivat von Flavonoiden natürlicher Herkunft steht.

4. Glucosylierter Komplex nach einem der Ansprüche 1 bis 3, **dadurch gekennzeichnet, dass** er ausgewählt wird unter:
- p-Retinoylphenylglucopyranosid,
- Diretinoyl-1,2-propanylglucopyranosid,
- Daidzinretinoat und
- Genistinretinoat.

5. Pharmazeutische oder kosmetische Zusammensetzung zur topischen Anwendung, **dadurch gekennzeichnet, dass** sie einen glucosylierten Komplex nach einem der Ansprüche 1 bis 4 kombiniert mit einem für die perkutane Verabreichung geeigneten Träger enthält.

6. Zusammensetzung nach Anspruch 5, **dadurch gekennzeichnet, dass** sie 0,001 bis 10 Gew.-%, vorzugsweise 0,01 bis 0,1 Gew.-% glucosylierten Komplex bezogen auf das Gesamtgewicht der Zusammensetzung enthält.

7. Zusammensetzung nach einem der Ansprüche 5 bis 6, **dadurch gekennzeichnet, dass** sie in Form einer Emulsion vorliegt.

8. Zusammensetzung nach einem der Ansprüche 5 bis 6, **dadurch gekennzeichnet, dass** sie in Form von Kügelchen, als Liposome, Nanokapseln oder Nanosphären vorliegt.

9. Verfahren zur Herstellung eines Komplexes nach einem der Ansprüche 1 bis 4, **dadurch gekennzeichnet, dass** man eine Verbindung der Formel (II) 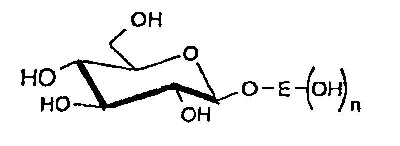 mit dem retinoischen Wirkstoff in Form eines Säurechlorids reagieren lässt.

10. Verfahren nach Anspruch 9, **dadurch gekennzeichnet, dass** die Verbindung der Formel II der folgenden Formel IIa entspricht:

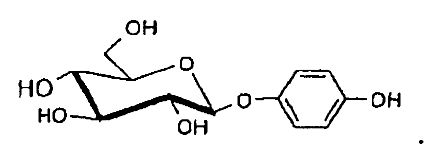

11. Verfahren nach Anspruch 9, **dadurch gekennzeichnet, dass** die Verbindung der Formel II der folgenden Formel IIb entspricht:

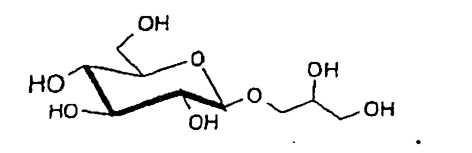

12. Verfahren nach einem der Ansprüche 9 bis 11, **dadurch gekennzeichnet, dass** das Säurechlorid Retinoylchlorid ist.

13. Verwendung einer Zusammensetzung nach einem der Ansprüche 5 bis 8 für die Herstellung eines Arzneimittels oder einer kosmetischen Zusammensetzung, welche(s), wenn es bzw. sie auf die Haut aufgetragen wird, dazu bestimmt ist, auf verzögerte Weise den retinoischen Wirkstoff auf verzögerte Weise ohne die Wirkung einer Anhäufung in den verschiedenen Schichten der Haut freizusetzen durch doppelte enzymatische Hydrolyse des Komplexes, zuerst vom Typ β-Glucocerebrosidase, welche zu der Hydrolyse zwischen der Glucose und der Abstandshaltergruppierung führt, dann vom Typ Esterase, welche zu der Hydrolyse zwischen der Abstandshaltergruppierung und dem Wirkstoff führt.

14. Verwendung nach Anspruch 13, **dadurch gekennzeichnet, dass** die Gruppierung E mit einer pharmazeutischen und/oder kosmetischen Aktivität ausgestattet ist.

15. Verwendung nach einem der Ansprüche 13 bis 14, **dadurch gekennzeichnet, dass** die Gruppierung E mit einer Hydratisierungs-, Depigmentierungs- und/oder antibakteriellen Aktivität ausgestattet ist.

## Claims

1. Ternary glucosyl complex, intended for percutaneous application, of formula (I) 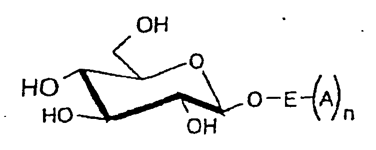 in which:
- E represents a group derived from L or D glycerol, hydroquinone or flavonoids,
- A represents a residue of a molecule of a retinoic active principle, linked to the spacer group via a carboxylate function -O-C(O)-,
- n = 1 or 2.

2. Glucosyl complex according to Claim 1, **characterized in that** the retinoic active principle is retinoic acid.

3. Glucosyl complex according to either of Claims 1 and 2, **characterized in that** the group E represents a derivative of flavonoids of natural origin.

4. Glucosyl complex according to one of Claims 1 to 3, **characterized in that** it is chosen from:
- para-retinoyl-phenyl-glucopyranoside,
- diretinoyl-1,2-propanyl-glucopyranoside,
- daidzin retinoate, and
- genistin retinoate.

5. Pharmaceutical or cosmetic composition for topical use, **characterized in that** it contains a glucosyl complex according to one of Claims 1 to 4, combined with a vehicle which is suitable for percutaneous administration.

6. Composition according to Claim 5, **characterized in that** it contains from 0.001% to 10% by weight and preferably 0.01% to 0.1% by weight of glucosyl complex relative to the total weight of the composition.

7. Composition according to either of Claims 5 and 6, **characterized in that** it is in the form of an emulsion.

8. Composition according to either of Claims 5 and 6, **characterized in that** it is in the form of spherules, for instance liposomes, nanocapsules or nanospheres.

9. Process for preparing a complex according to one of Claims 1 to 4, **characterized in that** a compound of formula (II) 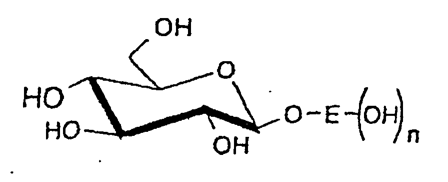 is reacted with the retinoic active principle in acid chloride form.

10. Process according to Claim 9, **characterized in that** the compound of formula II corresponds to formula IIa below:

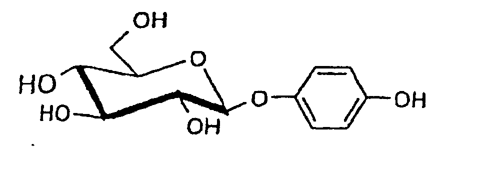

11. Process according to Claim 9, **characterized in that** the compound of formula II corresponds to formula IIb below:

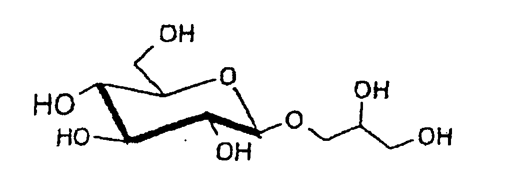

12. Process according to one of Claims 9 to 11, **characterized in that** the acid chloride is retinoyl chloride.

13. Use of a composition according to one of Claims 5 to 8, for the preparation of a medicament or a cosmetic composition intended, when it is applied to the skin, for releasing the retinoic active principle in a delayed manner without any effect of accumulation in the various layers of the skin, via double enzymatic hydrolysis of the complex, first of β-glucocerebrosidase type leading to hydrolysis between the glucose and the spacer group, and then of esterase type leading to hydrolysis between the spacer group and the active principle.

14. Use according to Claim 13, **characterized in that** the group E has a pharmaceutical and/or cosmetic activity.

15. Use according to either of Claims 13 and 14, **characterized in that** the group E has a moisturizing, depigmenting and/or antibacterial activity.
